(19) Europäisches Patentamt / European Patent Office / Office européen des brevets

(11) **EP 4 206 678 A1**

(12) **EUROPEAN PATENT APPLICATION**
published in accordance with Art. 153(4) EPC

(43) Date of publication:
**05.07.2023 Bulletin 2023/27**

(21) Application number: **21861663.9**

(22) Date of filing: **26.08.2021**

(51) International Patent Classification (IPC):
**G01N 35/08** (2006.01)  **G01N 37/00** (2006.01)

(52) Cooperative Patent Classification (CPC):
**G01N 35/08; G01N 37/00**

(86) International application number:
**PCT/JP2021/031326**

(87) International publication number:
**WO 2022/045241 (03.03.2022 Gazette 2022/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2020 JP 2020143865**

(71) Applicant: **Kyocera Corporation**
**Kyoto-shi Kyoto 612-8501 (JP)**

(72) Inventor: **IKEMOTO, Takeshi**
**Kyoto-shi, Kyoto 612-8501 (JP)**

(74) Representative: **TBK**
**Bavariaring 4-6**
**80336 München (DE)**

(54) **FLOW PASSAGE DEVICE AND LIQUID DELIVERY METHOD**

(57)  Measurement accuracy is improved. A flow channel device according to an aspect of the present disclosure includes a flow channel member having a flow channel, and at least a protrusion located in the flow channel and protruding in a downward direction from the upper surface of the flow channel, the downward direction being a gravitational direction when the flow channel device is in use.

FIG. 2

EP 4 206 678 A1

**Description**

TECHNICAL FIELD

The present disclosure relates to a flow channel device and a liquid feeding method for feeding a liquid using the flow channel device.

BACKGROUND OF INVENTION

Patent Document 1 discloses a micro total analysis chip including a primary flow channel for feeding a liquid and a plurality of divided flow channels which are branched plurally for dividing the liquid fed from the primary flow channel into a predetermined division ratio and feeding the liquid. Each of the plurality of divided flow channels which are branched plurally has a high flow channel resistance portion where a part of the flow channel is made narrower than front and rear parts of the flow channel to increase the flow channel resistance.

CITATION LIST

PATENT LITERATURE

Patent Document 1: JP 2008-122179 A

SUMMARY

In an aspect of the present disclosure, a flow channel device includes a flow channel member having a flow channel, and at least a protrusion located in the flow channel and protruding in a downward direction from an upper surface of the flow channel, the downward direction being a gravitational direction.

BRIEF DESCRIPTION OF THE DRAWINGS

FIG. 1 is a schematic view of a flow channel device according to a first embodiment of the present disclosure.
FIG. 2 is a cross-sectional view illustrating the structure of a flow channel member according to the first embodiment of the present disclosure.
FIG. 3 is a schematic view of the flow channel device according to a second embodiment of the present disclosure.
FIG. 4 is a cross-sectional view illustrating the structure of a flow channel member according to the second embodiment of the present disclosure.
FIG. 5 illustrates usage examples of the flow channel device according to the second embodiment of the present disclosure.
FIG. 6 illustrates a usage example of the flow channel device according to the second embodiment of the present disclosure.
FIG. 7 illustrates a variation of the flow channel member according to the second embodiment of the present disclosure.
FIG. 8 is a schematic view of a flow channel device according to a third embodiment of the present disclosure.
FIG. 9 is a schematic view of a flow channel device according to a fourth embodiment of the present disclosure.

DESCRIPTION OF EMBODIMENTS

First Embodiment

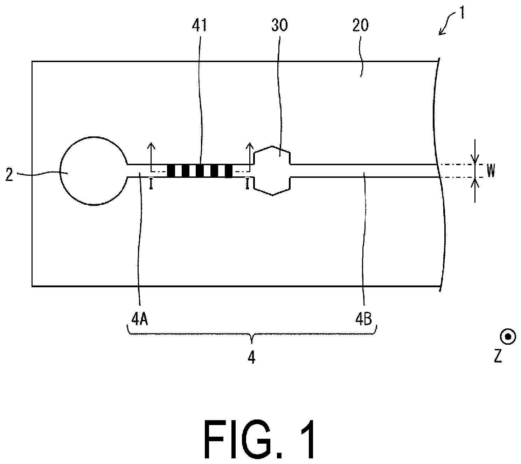

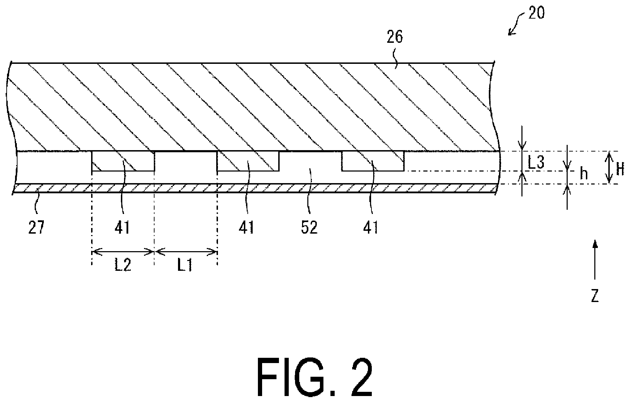

An embodiment of the present disclosure will be described in detail below. FIG. 1 is a schematic view of a flow channel device 1 according to a first embodiment of the present disclosure. FIG. 2 is a cross-sectional view illustrating the structure of a flow channel member 20 of the flow channel device 1, when viewed along arrow line I-I in FIG. 1. As illustrated in FIGs. 1 and 2, the flow channel device 1 includes a flow channel member 20 and a storage 2.

The flow channel member 20 is a member having a flow channel 4. In the present embodiment, the flow channel member 20 includes a base member 26 and a thin film 27 provided to face the base member 26, as illustrated in FIG. 2. The structure of the flow channel member 20 is not limited to the above structure, and the flow channel member 20 may be formed by making the base member 26 forming an upper surface of the flow channel 4 and the base member forming a lower surface of the flow channel 4 face each other and bonding them together.

The base member 26 may be made of a thermoplastic resin having a hydrophobic characteristic. A surface of the base member 26 having the hydrophobic characteristic can increase a contact angle of the liquid with respect to the surface of the base member 26. This effect will be described in detail later. Polycarbonate or cycloolefin polymers, for example, may be used as thermoplastic resins. The base member 26 includes a groove, which will be described later, on the surface on the side facing the thin film 27 to form the flow channel 4 and a detection unit 30 (predetermined location). The thin film 27 is made of a thermoplastic resin having a hydrophobic characteristic. The thin film 27 of the present embodiment may be made of a thermoplastic resin of the same material as the material of the base member 26. The base member 26 and the thin film 27 are thermally bonded to form the flow channel 4 and the detection unit 30. In the present embodiment, the flow channel 4 is formed such that the base member 26 is the upper surface and the thin film 27 is the lower surface when the flow channel device 1 is in use.

The storage 2 is a member that stores a liquid such as a reagent or a sample solution. Hereinafter, the term "liquid" is used as a generic expression for reagents and sample solutions. The storage 2 is not particularly limited but needs to feed a stored liquid to the flow channel 4, and may be, for example, a space in which the outflow of liquid is restrained due to a flow channel resistance or a space with a water head lower than the flow channel, or may be provided as a packaging container. The storage 2 need not be provided in the flow channel device 1, and a container for storing the liquid may be detachably connected to the flow channel 4 instead of the storage 2. Alternatively, an opening portion may be formed in the base member 26 of the flow channel device 1, and the liquid may be fed to the flow channel 4 by injecting the liquid from the opening portion.

The storage 2 of the present embodiment is provided as a packaging container storing a sample solution to be tested by the flow channel device 1, and may be bonded to the flow channel member 20 (more specifically, the base member 26) with an adhesive member. A well-known adhesive member may be used as the adhesive member. The storage 2 may be pressed with a rod (not illustrated) to feed the sample solution stored in the storage 2 to the detection unit 30, which will be described later, from the storage 2. The storage 2 need not be pressed with a rod, and any means capable of applying pressure, such as a finger or a board, can be used. The sample solution is, for example, a liquid containing a biological substance, but the type of substance contained in the sample solution is not particularly limited. Examples of the sample solution include urine, blood, sweat, saliva, or nasal discharge.

The detection unit 30 is a region for measuring a detection target substance contained in the sample solution. The detection unit 30 of the present embodiment is a chamber that uses a sensor (not illustrated) to measure an increase in weight caused by binding of an antigen contained in the sample solution to an antibody that is previously fixed in the detection unit 30.

The detection method for detecting a detection target substance in the detection unit 30 is not limited to the method described above. The detection method may be a method for measuring the intensity of fluorescence emitted by a fluorescent material directly or indirectly binding to the detection target substance, or a method for detecting the concentration of a product (such as a dye) directly or indirectly binding to the detection target substance.

The flow channel 4 includes a flow channel 4A and a flow channel 4B. The flow channel 4A guides a liquid stored in the storage 2 to the detection unit 30. The flow channel 4B discharges unwanted liquid from the detection unit 30. The flow channel 4B may be in an atmosphere non-exposed state (state in which the flow channel 4B is not exposed to atmospheric pressure), or in an atmosphere exposed state (state in which the flow channel 4B is exposed to atmospheric pressure). The channel 4B has a first end connected to the detection unit 30 and a second end connected to a waste storage (not illustrated) that stores a waste liquid and is in the atmosphere open state.

The flow channel device 1 includes at least one protrusion 41. The protrusion 41 is located in the flow channel 4 and protrudes downward from the upper surface of the channel 4 (that is, the base member 26). In the present embodiment, a plurality of protrusions 41 are connected to the base member 26 in the flow channel 4A. Specifically, the flow channel 4A includes five protrusions 41 connected along the direction in which the liquid is fed through the flow channel 4A. The protrusions 41 protrude in a downward direction from the upper surface of the flow channel 4A. As used herein, the downward direction means a gravitational direction (vertically downward direction) in a state in which the flow channel device 1 is in use. The state in which the flow channel device 1 is in use means a state in which the liquid is fed to the flow channel device 1. In each drawing of the present specification, the downward direction in a state in which the flow channel device 1 is in use corresponds to a negative Z-axis direction.

As described above, the flow channel device 1 of the present embodiment includes the flow channel member 20 including the flow channel 4 and the at least one protrusion 41 located in the flow channel 4 and protruding downward from the upper surface of the flow channel 4 (flow channel 4A). The flow channel 4 includes the flow channel 4A and the flow channel 4B. With the above structure of the flow channel device 1, when bubbles are entrained in the liquid being fed to the detection unit 30, and when the liquid reaches the protrusions 41, the bubbles float in a direction (vertically upward direction) opposite to the gravitational direction (vertically downward direction), and are blocked by the surface of the protrusions 41 that face the direction in which the sample solution flows. That is, the bubbles entrained in the liquid can be trapped by the protrusions 41. This improves the measurement accuracy of the detection unit 30.

The flow channel device 1 includes the plurality of protrusions 41 along the direction in which the flow channel 4 is fed. This increases the efficiency with which a gas entrained in the liquid is trapped. In the present embodiment, the flow channel device 1 includes five protrusions, but the number of the protrusions 41 is not limited to five and may be two or more.

The flow channel 4 has a width W at any position in the flow channel 4. The width W is the length of the flow channel in the direction perpendicular to the direction in which the flow channel extends, and can also be referred to as the thickness of the flow channel. However, the width W may be different depending on the position in the flow channel.

The protrusions 41 may function as a pressure barrier of the liquid to be fed. In other words, the protrusions 41 may function as a structural body for increasing a flow channel resistance. Specifically, a liquid is fed by passing through the protrusions 41 when a force greater than ΔP in the following equation is applied.

$$\text{Equation: } \Delta P = -2\gamma cos\theta \left[ (1/w) + (1/h) - (1/W) - (1/H) \right]$$

Definition of equation: w is a width of the micro flow channel, H is a height of the flow channel, h is a height of the micro flow channel, $\gamma$ is a surface tension of the liquid, and $\theta$ is a contact angle of a three-phase system. The height H of the flow channel means a height from the surface (tip) of the base member 26 to the thin film 27 in the vertical direction. The height h of the micro flow channel means a distance from the surface (tip) of the protrusions 41 to the thin film 27 in the vertical direction. The width w of the micro channel means a channel width of the flow channel where the protrusions 41 are located. The width w of the micro flow channel may be equal to the flow channel width W, or may be smaller than the flow channel width W. With the micro channel width w smaller than the channel width W, ΔP is larger, thus enhancing an effect of keeping the liquid stationary.

The plurality of protrusions 41 may be located along the direction in which the liquid is fed through the flow channel 4. The angle between the surface of each of the protrusions 41 facing the liquid flowing direction and a plane perpendicular to the liquid feeding direction and also perpendicular to the upper surface of the flow channel 4 may be five degrees. The external shape of the protrusions 41 can be set to any of various shapes. For example, each protrusion 41 may have a flat surface at its tip. In addition, the protrusions 41 may protrude in a sloped manner in a direction opposite to the liquid feeding direction, so that the liquid can move in the direction opposite to the liquid feeding direction. This makes it harder for the bubbles to overcome the protrusions 41 when lots of bubbles are entrained in the liquid and trapped by the protrusions 41.

The surfaces of the protrusions 41 may be made of a thermoplastic resin (material) having a hydrophobic characteristic. The surfaces of the protrusions 41 having a hydrophobic characteristic can increase the contact angle between the liquid and the protrusions 41. The contact angle between the liquid and the protrusions 41 may be 75 degrees or more, or may be 80 degrees or more. Increasing the contact angle makes it easier for the liquid to remain stationary.

As illustrated in FIG. 2, an interval L1 between adjacent protrusions 41 is greater than or equal to a length L2 of each protrusion in the liquid feeding direction. When the flow channel 4 is viewed in plan view, the length L2 can also be referred to as a length of the protrusion 41 protruding from the upper surface of the flow channel member 20 in a direction parallel to the direction in which the liquid is fed. The interval L1 between the adjacent protrusions 41 is less than three times the length in the width direction of the flow channel. When the flow channel 4 is viewed in plan view, the length of the protrusion 41 in the direction perpendicular to the liquid feeding direction (in other words, the width direction of the flow channel 4) is equal to the width W of the flow channel 4. This increases the efficiency with which entrained in the liquid are trapped.

As used herein, a ratio (h/H) of the channel height h in the region of the flow channel 4 where the protrusions 41 are connected to the channel height H in the region of the flow channel 4 where the protrusions 41 are not provided is defined as a "flow channel aspect ratio". In the present embodiment, the flow channel 4 has a flow channel aspect ratio of 1/2 (h : H = 0.5 : 1). That is, in the flow channel 4, the flow channel height h in the region where the protrusions 41 are connected is, but is not limited to, 0.5 times the flow channel height H in the region where the protrusions 41 are not provided.

In the present embodiment, the plurality of protrusions 41 located in the flow channel 4 makes the height of the flow channel 4 between adjacent protrusions 41 expand rapidly, thus decreasing a liquid feeding speed and making it easier for the liquid to remain stationary. Providing the plurality of protrusions 41 leads to a decrease in a length L3 of each protrusion compared with the structure including only one protrusion 41, because each protrusion 41 can have a smaller air bubble trapping function.

In the present embodiment, the interval L1 between adjacent protrusions is less than three times the length W in the width direction of the flow channel 4. Therefore, the flow channel device 1 in the present embodiment can be reduced in size compared with the structure in which the interval L1 between adjacent protrusions 41 is three times or more than the length W in the width direction of the flow channel 4.

In the present embodiment, the interval L1 between adjacent protrusions 41 is greater than or equal to the length L2 of the protrusions 41 protruding from the upper surface of the flow channel member. This allows the liquid to be fed to fill a first space formed between adjacent ones of the protrusions 41 and then be fed to a second space formed between subsequent protrusions 41, when the liquid is fed to pass through the region where the plurality of protrusions 41 are formed. In other words, setting the interval L1 to be greater than or equal to the length L2 can decrease the occurrence of gas entrainment that could occur if the liquid is fed into the second space before the first space is filled with the liquid.

In the flow channel device 1, the length of each protrusion 41 in the direction perpendicular to the direction in which the liquid is fed is equal to the width W of the flow channel 4. This increases the efficiency with which bubbles are trapped.

In the flow channel device 1, each of the protrusions 41 has a flat surface at its tip. This makes it easier to control the speed of the liquid in the region where the protrusions 41 are located.

In the flow channel device 1 according to the present embodiment, the flow channel member 20 includes the base member 26 and the thin film 27 provided to face the base member 26. By partially forming the surface constituting the flow channel with the thin film 27, the flow channel device 1 can be thinner than in a case where the entire flow channel is made of the base member 26. The thinner lower surface of the flow channel device 1 can increase heat transfer when heat is given to the liquid.

Second Embodiment

Another embodiment of the present disclosure will be described below. For convenience of description, a member having the same function as that of a member described in the embodiments described above is denoted by the same reference sign, and description thereof will not be repeated.

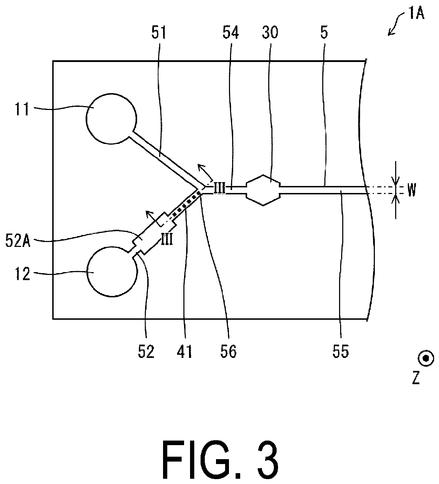

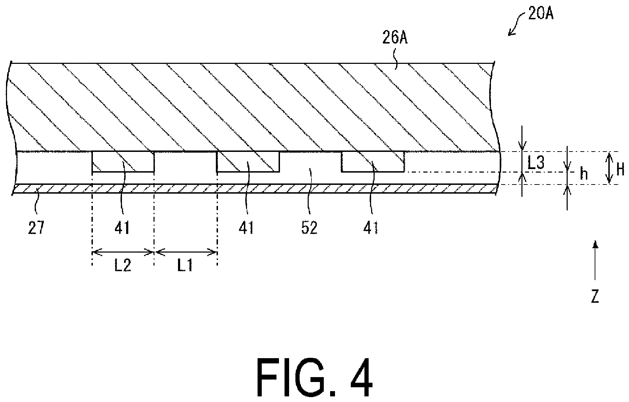

FIG. 3 is a schematic view of a flow channel device 1A according to the present embodiment. FIG. 4 is a cross-sectional view illustrating the structure of a flow channel member 20A in the flow channel device 1A, when viewed along arrow line III-III in FIG. 3. As illustrated in FIGs. 3 and 4, the flow channel device 1 includes a flow channel member 20A, a first storage 11, and a second storage 12. As illustrated in FIG. 4, the flow channel member 20A includes a base member 26A and the thin film 27 provided to face the base member 26A. The flow channel member 20A may include the basic structure, a material, and a variation the same as and/or similar to those of the flow channel member 20.

The base member 26A includes a groove formed on the surface on the side facing the thin film 27 to form a flow channel 5 which will be described later and the detection unit 30. The flow channel 5 and the detection unit 30 are formed by thermally bonding the base member 26A and the thin film 27. The base member 26A may include a material and a variation the same as and/or similar to those of the base member 26.

The first storage 11 and the second storage 12 are provided as sealing packaging containers, each containing a liquid, and may be bonded to the flow channel member 20A (more specifically, the base member 26A) with an adhesive member. Instead of the first and second storages 11, 12, three containers, each containing a liquid, may be detachably connected to the flow channel 4. Alternatively, the base member 26A may have two opening portions to feed a liquid to the flow channel 5 by injecting the liquid from each of the opening portions. The number of the storages is not limited to two, but may be more than one.

The types of the liquids stored in the first storage 11 and the second storage 12 are not particularly limited, and may be the same or may be different. In the present embodiment, the first storage 11 may store a buffer solution as the first liquid, and the second storage 12 may store a sample solution as the second liquid. In this case, the buffer solution stored in the first storage 11 is used to measure the baseline for the sample measurement.

In the present embodiment, the flow channel member 20A includes the flow channel 5. The liquids stored in the first storage 11 and the second storage 12 are fed to the flow channel 5 by being pressed with a rod (not illustrated). The first storage 11 and the second storage 12 may not be pressed with the rod, and any means capable of applying pressure, such as a finger or a board, can be used. The flow channel 5 includes a first flow channel 51, a second flow channel 52, a fourth flow channel 54, and a fifth flow channel 55. The first flow channel 51 and the second flow channel 52 are connected to the first storage 11 and the second storage 12, respectively. The first flow channel 51 and the second flow channel 52 are connected to a branching point 56. The fourth flow channel 54 has a first end side connected to the branching point 56 and a second end side connected to the detection unit 30. The fifth flow channel 55 is a flow channel for discharging a waste liquid from the detection unit 30. A first end side of the fifth flow channel 55 is connected to the detection unit 30 and a second end side is connected to a waste liquid storage that stores a waste liquid which is not illustrated.

The first liquid stored in the first storage 11 is guided through the first flow channel 51 and the fourth flow channel 54 to the detection unit 30. The second liquid stored in the second storage 12 is guided through the second flow channel 52 and the fourth flow channel 54 to the detection unit 30.

The first flow channel 51, the fourth flow channel 54, and the fifth flow channel 55 have a width W at any position in the flow channel. The width W is the length of the flow channel in the direction perpendicular to the direction in which the flow channel extends, and can also be referred to as the thickness of the flow channel. However, the width W may be different depending on the position in the flow channel. In contrast, the second flow channel 52 may have a wide portion 52A having a width greater than the width W. The wide portion 52A is filled with a gas, so that the gas filled in the wide portion 52A can push the liquid present in the fourth flow channel 54 and the detection unit 30 toward the fifth flow channel 55 when feeding the second liquid from the second storage 12. The gas filled in the wide portion 52A is not particularly limited. In the present embodiment, the wide portion 52A is filled with air. The wide portion 52A may include a printed reagent used for detecting reactions in the detection unit 30.

As illustrated in FIG. 3, in the second flow channel 52, the plurality of protrusions 41 are located along the liquid feeding direction of the second flow channel 52. The protrusions 41 are connected to the base member 26A of the flow channel member 20A and protrude downward from the upper surface of the second flow channel 52.

The surface of the flow channel member 20A facing the second flow channel 52 may have a hydrophobic characteristic. The surface having a hydrophobic characteristic has an effect of increasing the contact angle between the liquid and the flow channel. The contact angle may be 75 degrees or more, or 80 degrees or more. Such a large contact angle makes it easier for the liquid to remain stationary. In the flow channel device 1A, the surface of the flow channel member 20A having the hydrophobic characteristic and facing the second flow channel 52 can reduce inflow, or backflow, of the first liquid into the second flow channel 52.

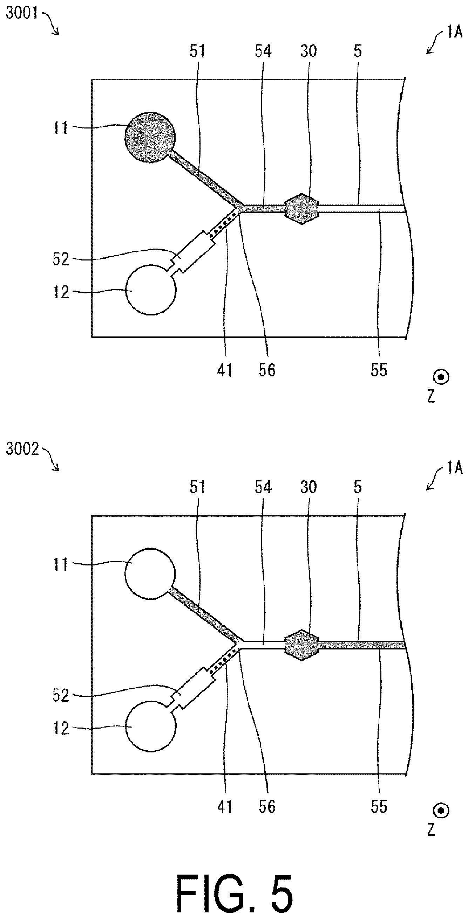

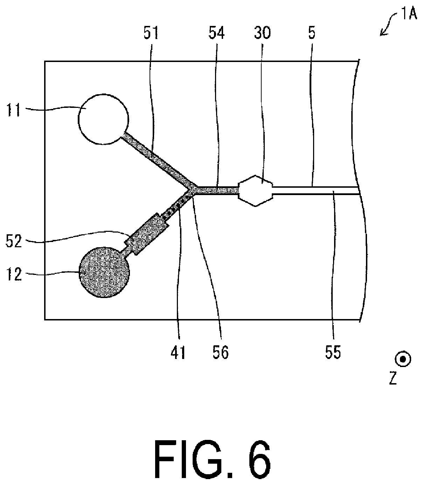

FIGs. 5 and 6 illustrate usage examples (liquid feeding methods) of the flow channel device 1A. FIG. 5 illustrates a state in which the first liquid is fed to the detection unit 30 as indicated by the reference sign 3001. FIG. 5 illustrates a state in which the first liquid is discharged from the detection unit 30 as indicated by the reference sign 3002. FIG. 6 illustrates a state in which the second liquid is fed to the detection unit 30.

As indicated by the reference sign 3001 in FIG. 5, the first storage 11 is pressed with a rod (not illustrated) to feed the buffer solution stored in the first storage 11 to the detection unit 30 via the first flow channel 51, the branching point 56, and the fourth flow channel 54 (first feeding step). At this time, since the protrusions 41 are provided at positions in the second flow channel 52 near the branching point 56, the buffer solution has a greater flow channel resistance in the second flow channel 52 at the positions where the protrusions 41 are provided. This decreases the backflow of the buffer solution toward the second flow channel 52 side. In other words, the protrusions 41 located in the second flow channel 52 can selectively determine the flow direction of the buffer solution in the flow channel device 1A.

As illustrated in FIG. 4, the second storage 12 is pressed with a rod (not illustrated) to feed the liquid stored in the second storage 12 to the detection unit 30 via the second flow channel 52, the branching point 56, and the fourth flow channel 54 (second feeding step). At this time, the presence of the protrusions 41 protruding downward from the upper surface of the second flow channel 52 allows the bubbles to be trapped when the bubbles are entrained in the liquid. This achieves appropriate measurement of the liquid in the detection unit 30.

The present embodiment need not include the wide portion 52A, but in the case where the wide portion 52A is included, as illustrated by the reference sign 3002 in FIG. 5, by pressing the second storage 12 with a rod (not illustrated), the air that has been present in the wide portion 52A before the pressing is pushed out to the detection unit 30 via the branching point 56. This increases the efficiency with which the first liquid present in the fourth flow channel 54 and the detection unit 30 is discharged through the fifth flow channel 55. In this case, the first liquid and the second liquid are separated across the air that has been present in the wide portion 52A, allowing a decrease in diffusion and mixture of the first liquid and the second liquid.

Since the present embodiment includes the plurality of protrusions 41 located in the second flow channel 52, the likelihood of the backflow of the first liquid to the second flow channel 52 side through the protrusions 41 can be reduced compared with the structure in which only one protrusion 41 is provided in the second flow channel 52.

Variation

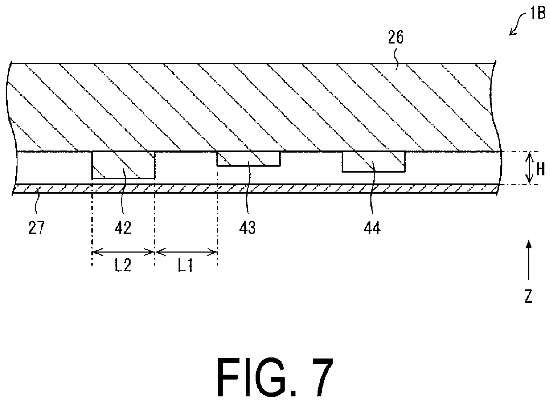

FIG. 7 illustrates a flow channel device 1B as a variation of the flow channel device 1 according to the first embodiment of the present disclosure. As illustrated in FIG. 7, the flow channel device 1B includes a protrusion 42, a protrusion 43, and a protrusion 44, instead of the plurality of protrusions 41 having the same protruding length in the first embodiment. The protrusion 42, the protrusion 43, and the protrusion 44 have different protruding lengths protruding from the upper surface of the flow channel. This provides different heights for regions where the protrusions 42, 43, and 44 are located, respectively, in the flow channel in which the plurality of protrusions 42 to 44 are formed in succession, thus providing different flow channel resistances for different regions. As a result, more types of liquids having, for example, different viscosities can be employed.

Third Embodiment

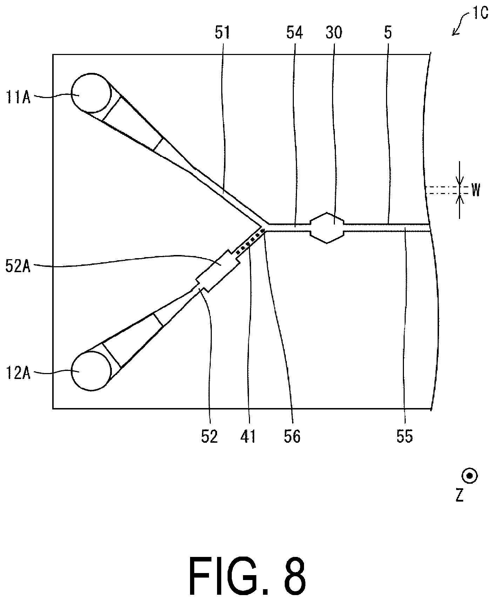

Another embodiment of the present disclosure will be described below. FIG. 8 is a schematic view of a flow channel device 1C according to a third embodiment of the present disclosure. As illustrated in FIG. 8, the flow channel device 1C includes a first injecting portion 11A and a second injecting portion 12A as the first storage and the second storage, respectively, instead of the sealing packaging containers storing liquids in the second embodiment. The first injecting portion 11A and the second injecting portion 12A are spaces for restraining the outflow of the liquid by the flow channel resistance. The first injecting portion 11A and the second injecting portion 12A are used to inject a reagent, a sample solution, or other liquids with a dropper or the like. The first injecting portion 11A and the second injecting portion 12A are opening portions formed in the base member 26.

In the flow channel device 1A of the second embodiment, the first storage 11 and the second storage 12 are pressed with a rod or the like to feed the liquids stored in the first storage 11 and the second storage 12 into the flow channel 5. In contrast, in the flow channel device 1C according to the present embodiment, air may be injected from the first injecting portion 11A and the second injecting portion 12A with a dropper or the like to feed the liquids stored in the first and second injecting portions 11A and 12A into the flow channel 5. This structure can also attain an effect which is the same as or similar to the effect of the flow channel device 1A in the second embodiment.

Fourth Embodiment

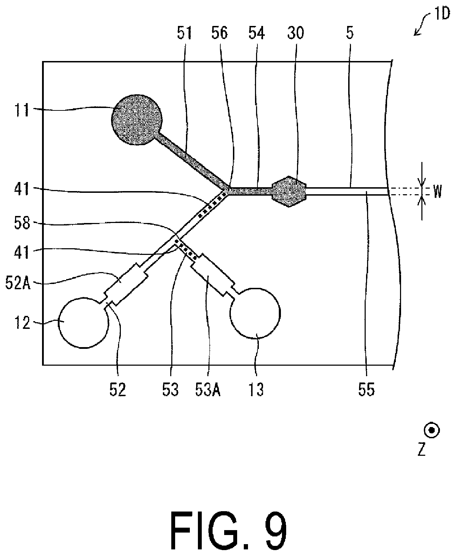

Another embodiment of the present disclosure will be described below. FIG. 9 is a schematic view of a flow channel device 1D according to a fourth embodiment of the present disclosure.

As illustrated in FIG. 9, the flow channel device 1D includes a third storage 13 and a third flow channel 53 in addition to the structure of the flow channel device 1A of the second embodiment. In the flow channel device 1D, the flow channel 5 includes the first flow channel 51, the second flow channel 52, the third flow channel 53, the fourth flow channel 54, and the fifth flow channel 55.

The structure of the third storage 13 is the same or similar to the structure of the first storage 11 and the second storage 12. The third storage 13 contains, for example, a buffer solution as a third liquid which is used, for example, to wash away the antigen (detection target substance) that has not been bound to the antibody in the detection unit 30.

The third flow channel 53 has one end connected to the third storage 13 and the other end connected to the second flow channel 52 at the branching point 58. The third liquid stored in the third storage 13 is guided to the detection unit 30 through the third flow channel 53, a portion of the second flow channel 52, and the fourth flow channel 54. The third flow channel 53 may have a wide portion 53A having a width greater than the width W. The wide portion 53A is filled with a gas, and the gas in the wide portion 53A can push out the liquid present in the second flow channel 52, the fourth flow channel 54, and the detection unit 30 toward the fifth flow channel 55 when the third liquid is fed from the third storage 13.

As illustrated in FIG. 9, the third flow channel 53 includes the plurality of protrusions 41 along the direction in which the liquid is fed to through the third flow channel 53. The interval between the plurality of protrusions 41 and the flow channel aspect ratio of the third flow channel 53 may be the same as or similar to the interval between the plurality of protrusions 41 and the flow channel aspect ratio of the second flow channel 52.

A usage example of the flow channel device 1D is described. In the flow channel device 1D, the first storage 11 is pressed with a rod (not illustrated) to feed the buffer solution stored in the first storage 11 to the detection unit 30 via the first flow channel 51, the branching point 56, and the fourth flow channel 54 (first feeding step). At this time, since the protrusions 41 are located in the second flow channel 52 near the branching point 56, the buffer solution is subject to an increase of the flow channel resistance of the second flow channel 52 at the positions where the protrusions 41 are provided. This decreases the backflow of the buffer solution toward the second flow channel 52 side.

Subsequently, the second storage 12 is pressed with a rod (not illustrated) to feed the liquid stored in the second storage 12 to the detection unit 30 via the second flow channel 52, the branching point 56, and the fourth flow channel 54 (second feeding step). At this time, the presence of the protrusions 41 protruding downward from the upper surface of the second flow channel 52 allows the bubbles to be trapped when the bubbles are entrained in the liquid. This achieves appropriate measurement of the liquid in the detection unit 30.

The protrusions 41 are provided in the third flow channel 53 near the branching point 58. This increases the flow channel resistance of the third flow channel 53 at the positions where the protrusions 41 are provided when the liquid is fed from the second storage 12 through the second flow channel 52. This decreases the backflow of the buffer solution toward the third flow channel 53 side.

Subsequently, the third storage 13 is pressed with a rod (not illustrated) to feed the liquid stored in the third storage 13 to the detection unit 30 via the third flow channel 53, the second flow channel 52, the branching point 56, and the fourth flow channel 54. Similar to the first storage 11 and the second storage 12, the third storage 13 may not be pressed with a rod, and any means capable of applying pressure, such as a finger or a board, can be used.

The present embodiment need not include the wide portion 53A, but in the case where the wide portion 53A is provided, by pressing the third storage 13 with a rod (not illustrated), the air that has been present in the wide portion 53A before the pressing is pushed out to the detection unit 30 via the branching points 56, 58. This increases the efficiency with which the second liquid present in the fourth flow channel 54 and the detection unit 30 is discharged through the fifth flow channel 55. In this case, the second liquid and the third liquid are separated across the air present in the wide portion 53A, thus decreasing the diffusion and mixture of the second liquid and the third liquid.

In the present embodiment, the plurality of protrusions 41 located in the third flow channel 53 make the height of the third flow channel 53 between adjacent protrusions 41 expand rapidly, thus decreasing the liquid feeding speed of the third liquid.

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make various variations or modifications based on the present disclosure. Note that these variations or modifications are included within the scope of the present disclosure.

REFERENCE SIGNS

    1, 1A to 1D Flow channel device
    2 Storage
    4, 5 Flow channel
    11 First storage
    12 Second storage
    11A First injecting portion (first storage)
    12A Second injecting portion (second storage)
    20, 20A Flow channel member
    26, 26A Base member
    27 Thin film
    30 Detection unit (predetermined location)
    41, 42, 43, 44 Protrusion
    56, 58 Branching point

**Claims**

1.  A flow channel device, comprising:

    a flow channel member having a flow channel; and
    at least a protrusion located in the flow channel and protruding in a downward direction from an upper surface of the flow channel, wherein
    the downward direction is a gravitational direction when the flow channel device is in use.

2.  The flow channel device according to claim 1, wherein
    a plurality of the protrusions is provided along a direction in which a liquid is fed through the flow channel.

3.  The flow channel device according to claim 1 or 2, wherein
    a surface of the protrusion is made of a material having a hydrophobic characteristic.

4.  The flow channel device according to any one of claims 1 to 3, wherein
    a surface facing the flow channel of the flow channel member has a hydrophobic characteristic.

5.  The flow channel device according to claim 2, wherein
    an interval between adjacent ones of the plurality of the protrusions is greater than or equal to a length of the protrusion protruding from the upper surface of the flow channel member in a direction parallel to the direction in which the liquid is fed when the flow channel is viewed in plan view.

6.  The flow channel device according to claim 2 or 5, wherein
    the interval between adjacent protrusions of the plurality of the protrusions is less than three times a length of the flow channel in a width direction.

7. The flow channel device according to claim 2, 5 or 6, wherein
each of the plurality of the protrusions has a different protruding length protruding from the upper surface of the flow channel member.

8. The flow channel device according to any one of claims 1 to 7, wherein
when the flow channel is viewed in plan view, the length of the protrusion in a direction perpendicular to the direction in which the liquid is fed is equal to the width of the flow channel.

9. The flow channel device according to any one of claims 1 to 8, wherein
the protrusion has a flat surface at its tip.

10. The flow channel device according to any one of claims 1 to 9, wherein
the flow channel member comprises a base member and a thin film provided to face the base member.

11. The flow channel device according to any one of claims 1 to 10, further comprising:
at least one storage connected to the flow channel and storing a liquid.

12. The flow channel device according to any one of claims 1 to 10, wherein
the flow channel member has a plurality of the flow channels and a branching point located in the plurality of the flow channels.

13. The flow channel device according to claim 12, wherein

a storage storing the liquid has a first storage that stores a first liquid and a second storage that stores a second liquid,
the plurality of the flow channels have a first flow channel that guides the first liquid stored in the first storage to a predetermined location, and a second flow channel that guides the second liquid stored in the second storage to a branching point with the first flow channel, and
the protrusion is located in at least part of the second flow channel.

14. A liquid feeding method for feeding a liquid using the flow channel device according to claim 13, comprising:

first feeding the first liquid stored in the first storage from the first storage to the predetermined location; and
subsequent to the first feeding, second feeding the second liquid stored in the second storage from the second storage to the predetermined location via the branching point.

FIG. 1

FIG. 2

FIG. 3

FIG. 4

FIG. 5

FIG. 6

FIG. 7

FIG. 8

FIG. 9

# EP 4 206 678 A1

## INTERNATIONAL SEARCH REPORT

| | International application No. |
|---|---|
| | **PCT/JP2021/031326** |

**A. CLASSIFICATION OF SUBJECT MATTER**

*G01N 35/08*(2006.01)i; *G01N 37/00*(2006.01)i
FI:    G01N35/08 A; G01N37/00 101

According to International Patent Classification (IPC) or to both national classification and IPC

**B. FIELDS SEARCHED**

Minimum documentation searched (classification system followed by classification symbols)

G01N35/00-35/10; G01N37/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2016-165704 A (PANASONIC INTELLECTUAL PROPERTY MANAGEMENT CO., LTD.) 15 September 2016 (2016-09-15) | 1-2, 5-6, 9 |
| | paragraphs [0124]-[0132], fig. 9 | |
| Y | paragraphs [0124]-[0132], fig. 9 | 3-4, 7-14 |
| X | WO 2007/125642 A1 (NIKKISO CO., LTD.) 08 November 2007 (2007-11-08) | 1-3, 5-6, 9 |
| | paragraphs [0026]-[0034], fig. 1-5 | |
| Y | paragraphs [0026]-[0034], fig. 1-5 | 3-4, 7-14 |
| Y | JP 2004-202336 A (FUJI ELECTRIC SYSTEMS CO., LTD.) 22 July 2004 (2004-07-22) paragraphs [0019]-[0024], fig.1, 2 | 4, 8-14 |
| Y | JP 2013-7592 A (KONICA MINOLTA ADVANCED LAYERS INC.) 10 January 2013 (2013-01-10) paragraphs [0061], [0062], fig. 4 | 7-14 |
| Y | WO 2006/109397 A1 (KONICA MINOLTA M & G, INC.) 19 October 2006 (2006-10-19) paragraphs [0062]-[0066], [0099]-[0135], fig. 2, 3, 7-9 | 11–14 |

| ✓ | Further documents are listed in the continuation of Box C. | ✓ | See patent family annex. |
|---|---|---|---|

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **08 October 2021** | **19 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
|---|
| **PCT/JP2021/031326** |

**C.    DOCUMENTS CONSIDERED TO BE RELEVANT**

| Category* | Citation of document, with indication, where appropriate, of the relevant passages | Relevant to claim No. |
|---|---|---|
| A | US 2006/0014269 A1 (STREIT, Wolfgang) 19 January 2006 (2006-01-19)<br>entire text, all drawings | 1-14 |

Form PCT/ISA/210 (second sheet) (January 2015)

**INTERNATIONAL SEARCH REPORT**
**Information on patent family members**

International application No.

**PCT/JP2021/031326**

| Patent document cited in search report | | | Publication date (day/month/year) | Patent family member(s) | | | Publication date (day/month/year) |
|---|---|---|---|---|---|---|---|
| JP | 2016-165704 | A | 15 September 2016 | (Family: none) | | | |
| WO | 2007/125642 | A1 | 08 November 2007 | US | 2009/0218704 | A1 | |
| | | | | paragraphs [0057]-[0065], fig. 1-5 | | | |
| | | | | EP | 2002883 | A2 | |
| JP | 2004-202336 | A | 22 July 2004 | (Family: none) | | | |
| JP | 2013-7592 | A | 10 January 2013 | (Family: none) | | | |
| WO | 2006/109397 | A1 | 19 October 2006 | US | 2006/0222572 | A1 | |
| | | | | paragraphs [0089]-[0093], [0127], [0162], fig. 2, 3, 7-9 | | | |
| | | | | EP | 1867385 | A1 | |
| | | | | CN | 101146607 | A | |
| US | 2006/0014269 | A1 | 19 January 2006 | EP | 1616619 | A1 | |
| | | | | DE | 202004011272 | U1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008122179 A